(19) Europäisches Patentamt
European Patent Office
Office européen des brevets

(11) Veröffentlichungsnummer: **0 015 452**
**B1**

# (12) EUROPÄISCHE PATENTSCHRIFT

(45) Veröffentlichungstag der Patentschrift: **20.01.82**

(51) Int. Cl.³: **C 07 D 253/06**, A 01 N 43/64 // C07D277/36

(21) Anmeldenummer: **80100875.6**

(22) Anmeldetag: **22.02.80**

(54) **6-Substituierte 3-Dimethylamino-4-methyl-1,2,4-triazin-5-(4H)-one, Verfahren zu ihrer Herstellung und ihre Verwendung als Herbizide.**

(30) Priorität: **07.03.79 DE 2908963**
**07.03.79 DE 2908964**
**22.09.79 DE 2938384**

(43) Veröffentlichungstag der Anmeldung: **17.09.80 Patentblatt 80/19**

(45) Bekanntmachung des Hinweises auf die Patenterteilung: **20.01.82 Patentblatt 82/3**

(84) Benannte Vertragsstaaten: **AT BE CH DE FR GB IT NL SE**

(56) Entgegenhaltungen:
**DE-A-2 165 554**
**FR-A-1 577 658**

(73) Patentinhaber: **BAYER AG, Zentralbereich Patente, Marken und Lizenzen, D-5090 Leverkusen 1, Bayerwerk (DE)**

(72) Erfinder: **Dickoré, Karlfried, Dr., Nicolai-Hartmann-Strasse 19, D-5090 Leverkusen (DE)**
Erfinder: **Sasse, Klaus, Dr., Pützweg 13, D-5060 Bergisch-Gladbach 2 (DE)**
Erfinder: **Eue, Ludwig, Dr., Paul-Klee-Strasse 36, D-5090 Leverkusen (DE)**
Erfinder: **Schmidt, Robert R., Dr., Hahnenweg 5, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# 6-Substituierte 3-Dimethylamino-4-methyl-1,2,4-triazin-5-(4H)-one, Verfahren zu ihrer Herstellung und ihre Verwendung als Herbizide

Die vorliegende Erfindung betrifft neue 6-substituierte Triazinon-Derivate, Verfahren zu ihrer Herstellung und ihre Verwendung als Herbizide.

Es ist bereits bekannt geworden, dass 1,2,4-Triazin-5-(4H)-one als Unkrautbekämpfungsmittel eingesetzt werden können. So ist zum Beispiel bekannt, dass bestimmte 3-amino-substituierte 4-Alkyl-6-phenyl-(oder -6-alkyl-) 1,2,4-triazin-5(4H)-one, z. B. 3-N-Morpholino-4-methyl-6-phenyl-1,2,4-triazin-5(4H)-on oder 3-Amino-4-methyl-6-phenyl-1,2,4-triazin-5(4H)-on, als Herbizide verwendbar sind (vgl. DE-A-1 670912). Aus FR-A-1 577658 und DE-A-2165554 sind weitere herbizid wirksame Triazinone dieses Types bekannt, so z. B. 3-Methylamino-4-methyl-6-isopropyl-1,2,4-triazin-5(4H)-on. Die Wirkung dieser Präparate ist jedoch in niedrigen Dosierungen nicht immer ausreichend.

Es wurden nunmehr die neuen 6-substituierten 3-Dimethylamino-4-methyl-1,2,4-triazin-5(4H)-one der Formel

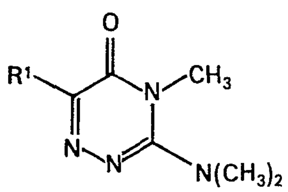

(I),

in welcher

$R^1$ für eine t-Butyl-, eine sec.-Butyl- oder eine Cyclohexylgruppe steht, aufgefunden.

Man erhält die Triazinone der Formel (I), wenn man

(a) ein 4-Methyl-3-alkylthio- (oder 3-aralkylthio)-triazin-5(4H)-on der allgemeinen Formel

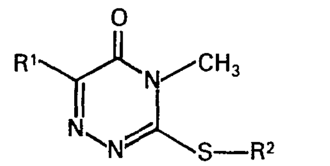

(II),

in welcher

$R^1$ die oben angegebene Bedeutung hat und $R^2$ für Alkyl oder Aralkyl steht, gegebenenfalls in Gegenwart eines Verdünnungsmittels und gegebenenfalls in Gegenwart einer niederen aliphatischen Carbonsäure mit Dimetyhlamin umsetzt, oder wenn man

(b) eine α-Ketocarbonsäure der Formel

$R^1$-CO-COOH (III),

in welcher

$R^1$ die oben angegebene Bedeutung hat,

in wässriger Lösung mit einem 3-Amino-1,1,2-trimethyl-guanidinium-Salz der Formel

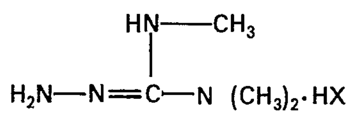

(IV),

in welcher

X für Cl, Br oder I steht,

umsetzt.

Die neuen Triazinone der Formel (I) weisen starke herbizide Eigenschaften auf.

Überraschenderweise zeigen die erfindungsgemässen neuen Triazinon-Derivate eine erheblich höhere herbizide Wirkung als die aus dem Stand der Technik vorbekannten 3-amino-substituierten 4-Alkyl-6-phenyl-(oder -6-alkyl-)-1,2,4-triazin-5-(4H)-one.

Verwendet man beispielsweise 6-tert.-Butyl-4-methyl-3-methylthio-1,2,4-triazin-5-(4H)-on und Dimethylamin als Ausgangsprodukte, so kann der Reaktionsablauf nach Verfahren (a) durch das folgende Formelschema wiedergegeben werden:

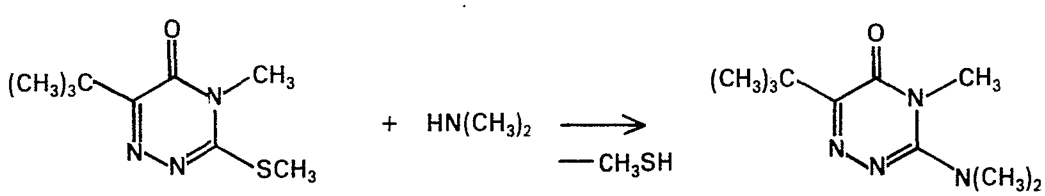


Verwendet man Cyclohexyl-glyoxylsäure und beispielsweise 3-Amino-1,1,2-trimethyl-guanidinium-hydroiodid als Ausgangsstoffe, so lässt sich der Reaktionsablauf nach Verfahren (b) durch das folgende Formelschema wiedergeben:

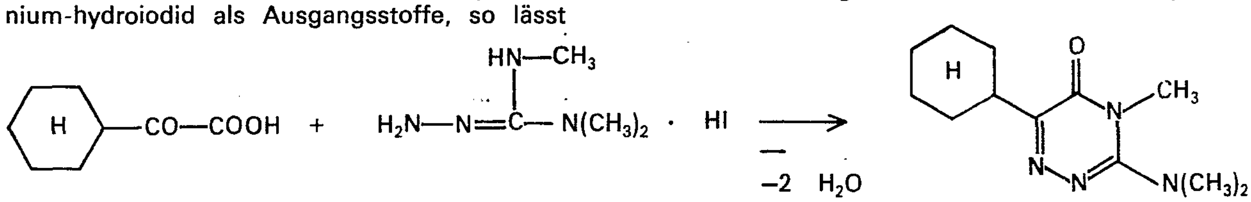

Die erfindungsgemäss verwendbaren Ausgangsstoffe sind grösstenteils bekannt. So wurde z. B. das für Verfahren (a) verwendbare 6-tert.-Butyl-4-methyl-3-methylthio-1,2,4-triazin-5(4H)-on (IIa) in 11%iger Ausbeute durch N-Methylierung von 6-tert.-Butyl-3-methylthio-1,2,4-triazin-5(4H)-on hergestellt (vgl. Z. Naturf. 31 B, 1122–1126 (1976)). Günstiger ist es jedoch, diese Verbindung durch S-Methylierung von 6-tert.-Butyl-4-methyl-5-oxo-3-thioxo-tetrahydro-1,2,4- (2H, 4H)-triazin (V) herzustellen (s. Herstellungsbeispiele).

Das für Verfahren (a) verwendbare 6-Cyclohexyl-4-methyl-3-methylthio-1,2,4-triazin-5(4H)-on (IIc) ist aus der DE-OS 1670912 bekannt. Diese Verbindung wird durch S.-Methylierung von 6-Cyclohexyl-4-methyl-5-oxo-3-thioxotetrahydro-1,2,4-(2H, 4H)-triazin (VI) hergestellt. – In analoger Weise kann das bisher noch nicht bekannte 6-sec.-Butyl-4-methyl-3-methylthio-1,2,4-triazin-5(4H)-on (II b) hergestellt werden. (s. Herstellungsbeispiele).

In ähnlicher Weise können auch andere 6-tert.-Butyl-, 6-sec.-Butyl- bzw. 6-Cyclohexyl-3-alkylthio- oder 3-aralkylthio-triazinone, beispielsweise die entsprechenden 3-Ethyl-thio, 3-Propylthio-, 3-Benzylthio- oder 3-(4-Chlorbenzylthio)-thriazinone, synthetisiert werden, welche in gleicher Weise als Ausgangsprodukte für Verfahren (a) geeignet sind.

Ein technisch durchführbares Verfahren zur Herstellung von (VI) beruht auf der Kondensation von Cyclohexanon mit N-Methylrhodanin zum Cyclohexyliden-N-methylrhodanin (VII), welches alkalisch zur 2-Mercapto-2-cyclohexyliden-essigsäure (VIII) hydrolysiert wird. Deren Kondensation mit 4-Methyl-thiosemicarbazid liefert (VI) (s. Herstellungsbeispiele).

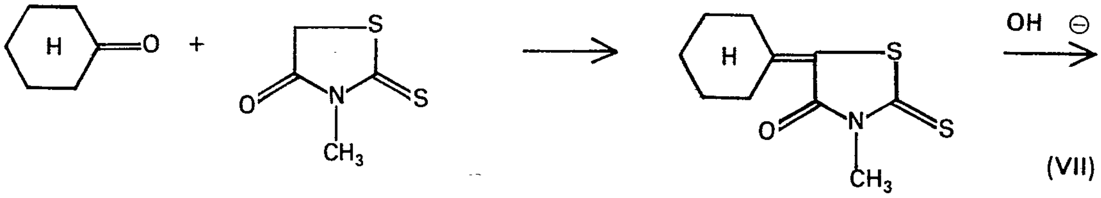


(VII)

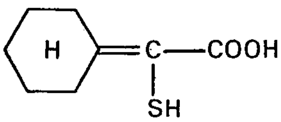

; (VIII) + $H_2N—NH—CS—NH—CH_3$

H ⊕

(VIII)

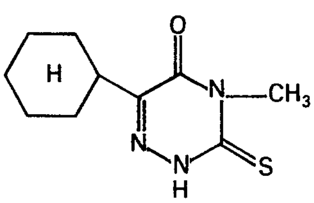

(VI)

3,3-Dimethyl-2-oxo-buttersäure (IIIa) ist bekannt. Die technische Herstellung dieser Verbindung kann z. B. nach dem in der DE-OS 2648300 angegebenen Verfahren erfolgen.

3-Methyl-2-oxo-valeriansäure (IIIb) ist gleichfalls bekannt (vgl. Monatshefte f. Chemie 26, 483–495 (1905); Beilsteins Handbuch der Organ. Chemie, 4. Aufl. (1921), Bd. 3. S. 690).

Cyclohexyl-glyoxylsäure (IIIc) ist ebenfalls bekannt. Die Herstellung dieser Verbindung kann z. B. nach dem in Liebigs Ann. Chem. 526, 47–58 (1936) angegebenen Verfahren erfolgen.

3-Amino-1,1,2-trimethyl-guanidinium-hydroiodid (IV, X=I) kann ebenfalls nach einem literaturbekannten Verfahren hergestellt werden (vgl. J. Org. Chem. 19, 180–1817 (1954)). Das entsprechende Hydrochlorid (IV, X=Cl) und Hydrobromid (IV, X=Br) können in analoger Weise hergestellt werden.

Bei Verfahren (a) kommen als Verdünnungsmittel alle inerten organischen Lösungsmittel in Frage. Hierzu gehören Kohlenwasserstoffe wie Toluol, Xylol; chlorierte aromatische Kohlenwasserstoffe wie Chlorbenzol, 1,2-Dichlorbenzol, 1,2,4-Trichlorbenzol; Ether wie Tetrahydrofuran, Dioxan; Alkohole wie Methanol, Ethanol, Propanol, Isopropanol; Amide wie N,N-Diemethylformamid, Tetramethylharnstoff oder Sulfoxide wie Dimethylsulfoxid. Vorzugsweise wird für die Umsetzung Isopropanol verwendet.

Die Reaktionstemperaturen können bei Verfahren (a) in einem grösseren Bereich variiert werden. Im allgemeinen arbeitet man bei Temperaturen zwischen etwa 20 und etwa 170 °C, vorzugsweise zwischen 60 und 90 °C.

Die Reaktion kann drucklos wie auch bei erhöhten Drucken ausgeführt werden.

Eine besonders vorteilhafte Ausführungsform des Verfahrens (a) besteht darin, dass man in Gegenwart mindestens der äquimolaren Menge einer niederen aliphatischen Carbonsäure arbeitet. Vorzugsweise wird hierfür Essigsäure verwendet. Dieses Verfahren gestattet es, mit relativ geringem Dimethylamin-Überschuss auszukommen. Bei dieser Ausführungsform kann die Reaktionsgeschwindigkeit durch Zusatz einer katalytischen Menge einer organischen Sulfonsäure erhöht werden. Vorzugsweise verwendet man hierfür p-Toluolsulfosäure.

Bei der Durchführung des erfindungsgemässen Verfahrens (a) nach dieser bevorzugten Verfahrensvariante setzt man auf 1 Mol des 3-Alkylthio-bzw. 3-Aralkylthio-triazinons der Formel (II) zweckmässigerweise 1 bis 2 Mol einer niederen aliphatischen Carbonsäure, 0,01 bis 0,05 Mol einer organischen Sulfosäure und 1 bis 2 Mol Dimethylamin ein, erhitzt bis zum Ende der Mercaptan-Abspaltung und arbeitet anschliessend auf. Dies kann z. B. in der Weise geschehen, dass man eindampft, den Rückstand mit überschüssiger wässriger Mineralsäure verrührt und ungelöste Verunreinigungen abtrennt. Durch Zusatz einer überschüssigen Menge einer Base, z. B. Ammoniak, wird das Reaktionsprodukt in hoher Reinheit ausgefällt. Verfahren (b) wird in wässriger Lösung durchgeführt. Zweckmässigerweise wird das Natriumsalz der α-Keto-carbonsäure (III) in wässriger Lösung vorgelegt, und nach Zugabe des 3-Amino-1,1,2-trimethylguanidinium-Salzes (IV) wird die Säure (III) durch Zusatz von Mineralsäure, beispielsweise Salzsäure, freigesetzt.

Die Reaktionstemperaturen können bei Verfahren (b) gleichfalls in einem grösseren Bereich variiert werden. Im allgemeinen arbeitet man bei Temperaturen zwischen etwa 50 und etwa 100 °C, vorzugsweise zwischen 65 und 90 °C.

Das Verfahren (b) wird im allgemeinen bei Normaldruck durchgeführt.

Bei der Durchführung des Verfahrens (b) setzt man auf 1 Mol einer wässrigen Lösung des Na-Salzes der α-Keto-carbonsäure (III) 1 bis 1,1 Mol eines 3-Amino-1,1,2-trimethyl-guanidinium-salzes (IV) ein und erhitzt nach Zugabe von Mineralsäure, beispielsweise Salzsäure, bis zum Ende der Reaktion. Die Aufarbeitung geschieht dann in der gleichen Weise wie für Verfahren (a) angegeben.

Die erfindungsgemässen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumwuchs. Ebenso können die Verbindungen zur Unkrautbekämpfung in Dauerkulturen, z. B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemässen Wirkstoffe zeigen insbesondere neben einer sehr guten Wirkung gegen grasartige Unkräuter auch eine herbizide Wirkung gegen breitblättrige Unkräuter, besonders auch gegen Galium-Arten. Ein selektiver Einsatz der erfindungsgemässen Wirkstoffe ist möglich, vorzugsweise in Mais, Erdnüssen, Sojabohnen, Baumwolle, Reis und anderen Getreidearten.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoffimprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage:

Aromaten, wie Xylol, Toluol, oder Alkylnapthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser.

Als fete Trägerstoffe kommen in Frage:

z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z. B. nichtionogene und aninonische Emulgatoren, wie Polyoxyäthylen-Fettsäure-Ester, Polyoxyäthlen-Fettalkohol-Äther, z. B. Alkylarylpolyglykol-Äther, Alkylsulfonate, Alkylsulfate, Arylsulfonate sowie Eiweisshydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-Metallphtalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 Gew.-%.

Die erfindungsgemässen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Giessen, Spritzen, Sprühen, Stäuben oder Streuen.

Die erfindungsgemässen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in grösseren Bereichen schwanken. Sie hängt im wesentlichen von der Art des gewünschten Effekts ab. Im allgemeinen liegen die Aufwandmengen zwischen 0,01 und 10 kg Wirkstoff pro ha, vorzugsweise zwischen 0,01 und 5 kg/ha.

Herstellungsbeispiele

A) 6-tert.-Butyl-3-dimethylamino-4-methyl-1,2,4-triazin-5(4H)-on (Ia) –nach Verfahren (a)

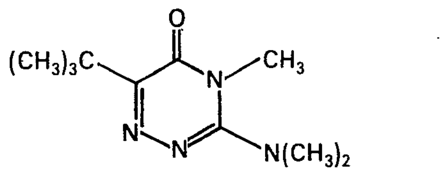

(Ia)

1850 g Essigsäure und 125 g p- Toluolsulfosäure werden in 15 Liter Isopropanol vorgelegt. Unter Kühlung leitet man 1800 g Dimethylamin ein, gibt dann 5250 g (24,65 Mol) 6-tert.-Butyl-4-methyl-3-methylthio-1,2,4-triazin-5(4H)-on zu und erhitzt bis zum Ende der Methylmercaptan-Entwicklung unter Rückfluss. Sodann wird die Hauptmenge des Lösungsmittels bei einer Innentemperatur von 80–90 °C unter Durchleiten von Stickstoff abdestilliert.

Den öligen Rückstand verrührt man bei 0–5 °C mit 25 Liter einer 5%igen Salzsäure und 2 Liter Chloroform. Die organische Phase wird abgetrennt und verworfen. Zur wässrigen Phase gibt man unter Kühlung 3 Liter 25%ige Ammoniak-Lösung, wobei das Reaktionsprodukt kristallin ausfällt. Man saugt bei 0 °C ab, wäscht mit wenig Eiswasser und trocknet bei 50 °C.

Man erhält 4438 g (86% d.Th.) der oben bezeichneten Verbindung (Ia) vom Schmp. 89–91 °C. Die gas-chromatographisch bestimmte Reinheit liegt über 99%. Die Verbindung ist destillierbar: Kp. 115 °C/0,03 mbar.

B) 6-sec.-Butyl-3-dimethylamino-4-methyl-1,2,4-triazin-5(4H)-on (Ib) – nach Verfahren (a)

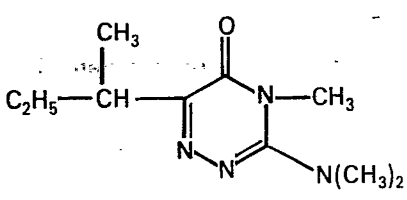

(Ib)

1065 g (5 Mol) 6-sec.-Butyl-4-methyl-3-methylthio-1,2,4-triazin-5(4H)-on, 375 g Essigsäure und 25 g p-Toluolsulfosäure werden in 3 Liter Isopropanol vorgelegt. Man leitet ohne Kühlung 360 g (8 Mol) Dimethylamin ein, kocht 6 Stunden unter Rückfluss und leitet danach in die siedende Mischung innerhalb von 6 Stunden nochmals 225 g (5 Mol) Dimethylamin ein.

Bei 90–100 °C wird die Hauptmenge des Lösungsmittels unter Durchleiten von Stickstoff zunächst bei Normaldruck abdestilliert; danach entfernt man bei 20 mbar und einer Bad-Temperatur von 100 °C Restmengen des Lösungsmittels.

Den öligen Rückstand verrührt man bei 0–5 °C mit 6 Liter Eiswasser und 460 ml 37%iger Salzsäure, gibt 400 ml Chloroform zu und trennt nach kurzem Durchrühren die Phasen. Die organische Phase wird verworfen, zur wässrigen Phase gibt man bei 10–20 °C unter Rühren 2 Liter 25%ige Ammoniak-Lösung, nimmt das abgeschiedene Öl in 400 ml Chloroform auf, trennt die Phasen, rührt die wässrige Phase noch einmal mit 400 ml Chloroform aus und destilliert den Eindampfrückstand der vereinigten Chloroform-Lösungen im Hochvakuum.

Man erhält 892 g (85% d.Th.) der oben bezeichneten Verbindung (Ib) vom Kp. 131 °C/0,15 mbar und mit dem Brechungsindex $n_D^{20} = 1,5324$.

Die gas-chromatographisch bestimmte Reinheit beträgt 99%.

C) 6-Cyclohexyl-3-dimethylamino-4-methyl-1,2,4-triazin-5(4H)-on (Ic)- nach Verfahren (a)

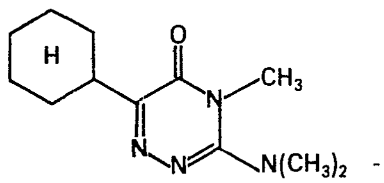

(1c)

1850 g Essigsäureund 125 g Toluolsulfosäure werden in 15 Liter Isopropanol vorgelegt. Unter Kühlung leitet man 1800 g Dimethylamin ein, gibt dann 5850 g (24,48 Mol) 6-Cyclohexyl-4-methyl-3-methylthio-1,2,4-triazin-5(4H)-on zu und erhitzt bis zum Ende der Methylmercaptan-Entwicklung unter Rückfluss. Sodann wird die Hauptmenge des Lösungsmittels bei einer Innentemperatur von 80–90 °C unter Durchleiten von Stickstoff abdestilliert.

Den öligen Rückstand verrührt man bei 0–5 °C mit 25 Liter einer 5%igen Salzsäure und 2 Liter Chloroform. Die organische Phase wird abgetrennt und verworfen. Zur wässrigen Phase gibt man unter Kühlung 3 Liter 25 %ige Ammoniak-Lösung, wobei das Reaktionsprodukt kristallin ausfällt. Man saugt bei 0 °C ab, wäscht mit wenig Eiswasser und trocknet bei 50 °C.

Man erhält 4740 g (82% d.Th.) der oben bezeichneten Verbindung (Ic) vom Schmp. 104–106 °C.

D) Ausgangsprodukte

a) 6-tert.-Butyl-4-methyl-5-oxo-3-thioxo-tetrahydro-1,2,4-(2H,4H)-triazin (V)

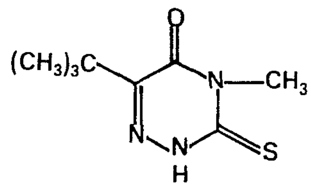

(V)

Man löst 4725 g (45 Mol) 4-Methyl-thiosemicarbazid in 25 Liter Wasser und 6 Liter 35 %iger Salzsäure, gibt unter Rühren 92 Liter einer wässrigen Lösung des Na-Salzes der 3,3-Dimethyl-2-oxo-buttersäure zu, die 46 Mol enthält, und kocht 10 Stunden unter Rückfluss. Man saugt bei 50 °C ab, wäscht mit Wasser und trocknet bei 80 °C. Man erhält 7340 g (82 % d.Th.) der Verbindung (V) vom Schmp. 214 °C.

b) 6-tert.-Butyl-4-methyl-3-methylthio-1,2,4-triazin-5-(4H)-on (IIa)

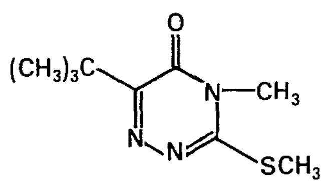

(IIa)

Zu einer Lösung von 1145 g NaOH in 57 Liter Wasser gibt man 30 ml 3-Benzyl-4-hydroxy-biphenyl-polyglykolether als Emulgator sowie 5690 g (28,6 Mol) 6-tert.-Butyl-4-methyl-5-oxo-3-thioxo-tetrahydro-1,2,4(2H,4H)-triazin. Man tropft 4302 g (30,3 Mol) Methyliodid zu (oder leitet 2900 g Methylbromid ein) und rührt nach, bis die schwach exotherme Reaktion beendet und ein pH-Wert von 7–8 erreicht ist. Nach Absaugen, Waschen mit Wasser und Trocknen bei 50 °C erhält man 5810 g (95% d.Th.) der Verbindung (IIa) vom Schmp. 90–100 °C. Das Rohprodukt ist für die weitere Umsetzung hinreichend rein. Eine aus Methanol oder Petrolether umkristallisierte Probe schmilzt bei 104 – 105 °C.

c) 6-sec.-Butyl-4-methyl-5-oxo-3-thioxo-tetrahydro-1,2,4(2H,4H)-triazin (IX)

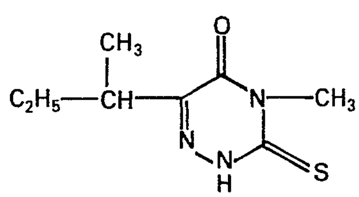

(IX)

Man löst 525 g (5 Mol) 4-Methyl-thiosemicarbazid in 2 Liter Waser und 600 ml 37 %iger Salzsäure und gibt bei 95–100 °C eine Lösung von 760 g (5 Mol) des Na-Salzes der 3-Methyl-2-oxo-valeriansäure in 1,5 Liter Wasser rasch zu. Man kocht 10 Stunden unter Rückfluss, lässt unter Rühren abkühlen, saugt ab und wäscht mit 2 Liter Wasser. Das feuchte Rohprodukt wird sodann mit 1 Liter

Methanol bei -20 °C gerührt, bei -20 °C abgesaugt und mit Menthanol von -70 °C gewaschen. Man erhält nach Trocknen bei 80 °C 697 g (70 % d.Th.) der Verbindung (IX) vom Schmp. 117-119 °C. Eine aus Methanol umkristallisierte Probe schmilzt bei der gleichen Temperatur.

d) 6-sec.-Butyl-4-methyl-3-methylthio-1,2,4-triazin-5(4H)-on (IIb)

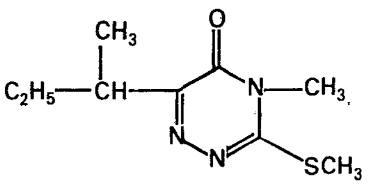

(IIb).

Zu einer Lösung von 202 g NaOH in 5 Liter Wasser gibt man 1 ml 3-Benzyl-4-hydroxy-biphenyl-polyglykolether als Emulgator sowie 995 g (5 Mol) 6-sec.-Butyl-4-methyl-5-oxo-3-thioxo-tetrahydro-1,2,4 (2H,4H)-triazin (IX). Man tropft 724 g (5,1 Mol) Methyliodid zu und rührt nach, bis die schwach exotherme Reaktion beendet und ein pH-Wert von 7-8 erreicht ist. Nach Absaugen, Waschen mit Wasser und Trocknen bei 30 °C im Vakuum erhält man 907 g (85% d.Th.) der Verbindung (IIb) vom Schmp. 45-50 °C. Das Rohprodukt ist für die weitere Umsetzung hinreichend rein. Eine aus Pentan umkristallisierte Probe schmilzt bei 54-55 °C.

e) Cyclohexyliden-N-methyl-rhodanin (VII)

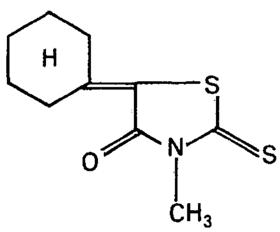

(VII)

Man suspendiert 4807 g (32,7 Mol) N-Methyl-rhodanin in 19,6 Liter Methanol, gibt 6420 g (65,4 Mol) Cyclohexanon zu und erwärmt unter Rühren auf 50 °C. Sodann werden ohne weitere Heizung 570 g (6,54 Mol) Morpholin zugetropft, wobei das Ausgangsprodukt in Lösung geht und eine exotherme Reaktion abläuft. Nach kurzer Zeit beginnt (VII) in hellgelben Kristallen auszufallen. Nach Ende der exothermen Reaktion kocht man noch eine Stunde unter Rückfluss, saugt bei 20 °C ab und wäscht mehrfach mit Methanol. Man erhält 7190 g (97% d.Th.) der Verbindung (VII) vom Schmp. 111-113 °C.

f) 6-Cyclohexyl-4-methyl-5-oxo-3-thioxo-tetrahydro-1,2,4(2H,4H)-triazin (VI)

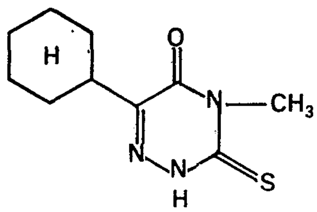

(VI)

Man suspendiert 7180 g (31,6 Mol) Cyclohexyliden-N-methyl-rhodanin in einer Auflösung von 6326 g Natriumhydroxid in 60 Litern Wasser und rührt 10 Stunden bei 60 °C. nach dieser Zeit ist nahezu alles gelöst. Ungelöste Reste werden durch Ausrühren mit 2 Litern Chloroform entfernt. Nach Abtrennen der organischen Phase trägt man in die so erhaltene wässrige Lösung des Na-Salzes der 2-Mercapto-2-cyclohexyliden-essigsäure (VIII) 3320 g (31,6 Mol) 4-Methyl-thiosemicarbazid ein, heizt auf 80 °C und tropft 19,0 Liter 32 %ige Salzsäure ein. Unter $H_2S$-Entwicklung erfolgt Kondensation zu (VI). Man saugt das Reaktionsprodukt bei 60 °C ab, wäscht mehrfach mit Wasser und erhält nach dem Trocknen bei 80 °C 5830 g (82% d.Th.) der oben bezeichneten Verbindung vom Schmp. 173-176 °C. Eine aus Chlorbenzol umkristallisierte Probe schmilzt bei 186 %°C.

g) 6-Cyclohexyl-4-methyl-3-methylthio-1,2,4-triazin-5-(4H)-on (IIc)

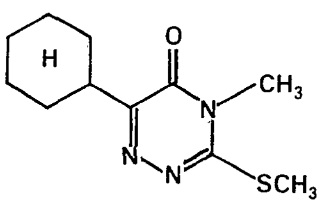

(IIc)

Zu einer Lösung von 1033 g NaOH in 52 Liter Wasser gibt man 30 ml 3-Benzyl-4-hydroxy-biphenyl-polyglykolether als Emulgator sowie 5805 g (25,8 Mol) 6-Cyclohexyl-4-methyl-5-oxo-3-thioxo-tetrahydro-1,2,4(2H,4H)-triazin. Man tropft 3881 g (27,3 Mol) Methyliodid zu (oder leitet 2620 g Methylbromid ein) und rührt nach, bis die schwach exotherme Reaktion beendet und ein pH-Wert von 7-8 erreicht ist. Nach Absaugen, Waschen mit Wasser und Trocknen bei 50 °C erhält man 5860 g (95% d.Th.) der Verbindung (IIc) vom Schmp. 84-88 °C. Das Rohprodukt ist für die weitere Umsetzung hinreichend rein. Eine aus Methanol oder Petrolether umkristallisierte Probe schmilzt bei 90 °C.

Verwendungsbeispiele

Beispiel A

Pre-emergence-Test

Lösungsmittel: 5Gewichtsteile Aceton

Emulgator: 1 Gewichtsteil Alkylarylpolyglycoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeutet:

0% = keine Wirkung (wie unbehandelte Kontrolle)
100% = totale Vernichtung

In diesem Test zeigen die Wirkstoffe (Ia), (Ib) und (Ic) eine ausgezeichnete Wirkung.

Beispiel B
Post-emergence-Test
Lösungsmittel: 5 Gewichtsteile Aceton
Emulgator: 1 Gewichtsteil Alkylarylpolyglycoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, dass die jeweils gewünschte Wirkstoffmenge pro Flächeneinheit ausgebracht wird. Die Konzentration der Spritzbrühe wird so gewählt, dass in 2000 l Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Auch in diesem Test zeigen die Wirkstoffe (Ia), (Ib) und (Ic) eine hervorragende Wirkung.

**Patentansprüche:**

1. 6-Substituierte 3-Dimethylamino-4-methyl-1,2,4-triazin-5(4H)-one der Formel

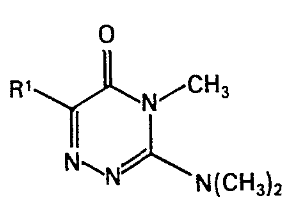

(I),

in welcher
$R^1$ für eine t-Butyl-, eine sec.-Butyl- oder eine Cyclohexylgruppe steht.

2. Verfahren zur Herstellung von 6-substituierten 3-Dimethylamino-4-methyl-1,2,4-triazin-5(4H)-onen der Formel

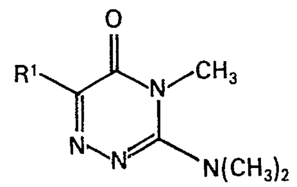

(I),

in welcher
$R^1$ für eine t-Butyl-, eine sec.-Butyl- oder eine Cyclohexylgruppe steht,
dadurch gekennzeichnet, dass man
(a) ein 4-Methyl-3-alkylthio-(oder 3-aralkylthio)-triazin-5(4H)-on der allgemeinen Formel

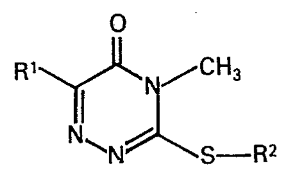

(II),

in welcher
$R^1$ die oben angegebene Bedeutung hat und
$R^2$ für Alkyl oder Aralkyl steht,
gegebenenfalls in Gegenwart eines Verdünungsmittels und gegebenenfalls in Gegenwart einer niederen aliphatischen Carbonsäure mit Dimethylamin umsetzt, oder dass man

(b) eine α-Ketocarbonsäure der Formel

$R^1$-CO-COOH (III),

in welcher
$R^1$ die oben angebene Bedeutung hat,
in wässriger Lösung mit einem 3-Amino-1,1,2-trimethyl-guanidinium-Salz der Formel

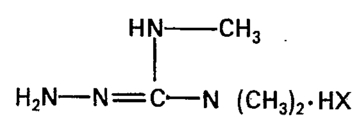

(IV),

in welcher
X für Cl, Br oder I steht, umsetzt.

3. Herbizide Mittel, gekennzeichnet durch einen Gehalt an einem 6-substituierten 3-Dimethylamino-4-methyl-1,2,4-triazin-5(4H)-onen der Formel (I) gemäss Ansprüchen 1 oder 2.

4. Verwendung von 6-substituierten 3-Dimethylamino-4-methyl-1,2,4-triazin-5(4H)-onen der Formel (I) gemäss Ansprüchen 1 oder 2 zur Bekämpfung von Pflanzenwachstum.

5. Verfahren zur Herstellung von herbiziden Mitteln, dadurch gekennzeichnet, dass man 6-substituierte 3-Dimethylamino-4-methyl-1,2,4-triazin-5(4H)-one der Formel (I) gemäss Ansprüchen 1 oder 2 mit Streckmitteln und/oder oberflächenaktiven Mitteln vermischt.

## Claims

1. 6-Substituted 3-dimethylamino-4-methyl-1,2,4-triazin-5(4H)-ones of the formula

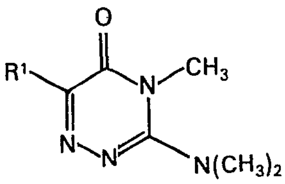

(I),

in which
$R^1$ represents a t-butyl group, a sec.-butyl group or a cyclohexyl group.

2. Process for the preparation of 6-substituted 3-dimethylamino-4-methyl-1,2,4-triazin-5(4H)-ones of the formula

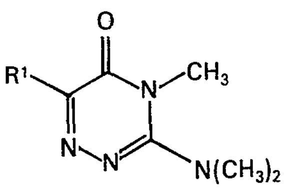

(I),

in which
$R^1$ represents a t-butyl group, a sec.-butyl group or a cyclohexyl group,
characterised in that

a) a 4-methyl-3-alkylthio-(or 3-aralkylthio)-triazin-5(4H)-one of the general formula

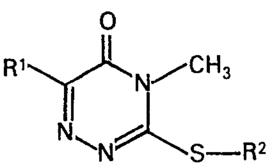

(II),

in which
$R^1$ has the meaning indicated above and
$R^2$ represents alkyl or aralkyl,
is reacted with dimethylamine, if appropriate in the presence of a diluent and if appropriate in the presence of a lower aliphatic carboxylic acid, or in that

b) an α-ketocarboxylic acid of the formula

$R^1$-CO-COOH (III),

in which
$R^1$ has the meaning indicated above,
is reacted with a 3-amino-1,1,2-trimethylguanidinium salt of the formula

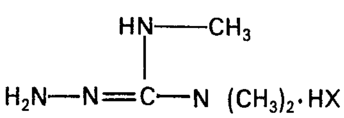

(IV),

in which
X represents Cl, Br or I,
in aqueous solution.

3. Herbicidal agents, characterised in that they contain a 6-substituted 3-dimethylamino-4-methyl-1,2,4-triazin-5(4H)-one of the formula (I) according to Claims 1 or 2.

4. Use of 6-substituted 3-dimethylamino-4-methyl-1,2,4-triazine-5(4H)-ones of the formula (I) according to Claims 1 or 2 for combating plant growth.

5. Process for the preparation of herbicidal agents, characterised in that 6-substituted 3-dimethylamino-4-methyl-1,2,4-triazin-5(4H)-ones of the formula (I) according to Claims 1 or 2 are mixed with extenders and/or surface-active agents.

## Revendications

1. 3-diméthylamino-4-méthyl-1,2,4-triazine-5(4H)-ones substituées en posititon 6, de formule

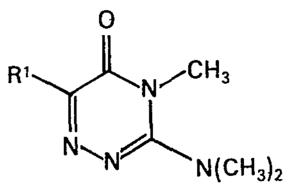

(I),

dans laquelle
$R^1$ représente un groupe tert-butyle, sec-butyle ou cyclohexyle.

2. Procédé de préparation des 3-diméthylamino-4-méthyl-1,2,4-triazine-5(4H)-ones substituées en position 6, de formule

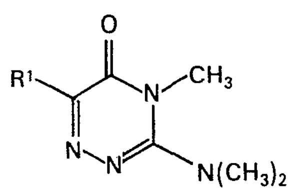

(I),

dans laquelle
$R^1$ représente un groupe tert-butyle, sec-butyle ou cyclohexyle, caractérisé en ce que:

(a) on fait réagir une 4-méthyl-3-alkylthio-(ou 3-aralkylthio)-triazine-5(4H)-one de formule générale

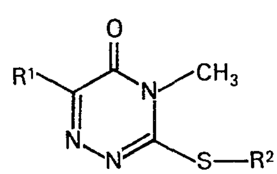

(II),

dans laquelle
$R^1$ a la signification indiquée ci-dessus-et
$R^2$ représente un groupe alkyle ou aralkyle,
éventuellement en présence d'un diluant et éventuellement en présence d'un acide carboxylique aliphatique inférieur, avec la diméthylamine, ou bien

(b) on fait réagir un acide α-cétocarboxylique de formule

$R^1$-CO-COOH (III)

dans laquelle $R^1$ a la signification indiquée ci-dessus, en solution aqueuse, avec un sel de 3-amino-1,1,2-triméthyl-guanidinium de formule

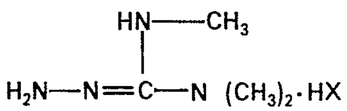

(IV),

dans laquelle X représente Cl, Br ou I.

3. Produit herbicide caractérisé en ce qu'il contient une 3-diméthylamino-4-méthyl-1,2,4-triazine-5(4H)-one substituée en position 6, de formule (I) selon les revendications 1 ou 2.

4. Utilisation des 3-diméthylamino-4-méthyl-1,2,4-triazine-5(4H)-ones substituées en position 6, de formule (I) selon les revendications 1 ou 2, pour combattre la croissance des végétaux.

5. Procédé de préparation des produits herbicides caractérisé en ce que l'on mélange des 3-diméthylamino-4-méthyl-1,2,4-triazine-5(4H)-ones substituées en position 6, de formule (I) selon les revendications 1 ou 2, avec des diluants et/ou de agents tensio-actifs.